# EUROPEAN PATENT APPLICATION

(11) **EP 3 905 255 A1**
(43) Date of publication of application: **03.11.2021**
(21) Application number: 20171632.1
(22) Date of filing: 27.04.2020
(51) Int. Cl.: G16H 10/20, G16H 50/70

(54) **MAPPING A PATIENT TO CLINICAL TRIALS FOR PATIENT SPECIFIC CLINICAL DECISION SUPPORT**

(71) Applicant: Siemens Healthcare GmbH, 91052 Erlangen (DE)
(72) Inventor: SHARMA, Puneet, Princeton Junction, NJ New Jersey 08550 (US); NEUMANN, Dominik, 91052 Erlangen (DE)
(74) Representative: EIP

(57) **Abstract**

Systems and methods for mapping a patient to one or more clinical trials are provided. Patient data of a patient is received and encoded into a patient model of the patient. Synthetic patients are generated for each clinical trial in a set of clinical trials based on characteristics of participants in that clinical trial. The patient model of the patient is compared to each of the synthetic patients to identify synthetic patients matching the patient model. The patient is mapped to one or more clinical trials in the set of clinical trials based on the matching synthetic patients.

## Description

### TECHNICAL FIELD

The present invention relates generally to patient specific clinical decision support, and in particular to automatically mapping a patient to a clinical trial for patient specific clinical decision support.

### BACKGROUND

Clinical trials are typically performed to evaluate the safety and efficacy of prospective treatments. Examples of such prospective treatments include drugs, medical devices, and medical interventions. However, most physicians do not incorporate results of clinical trials until clinical guidelines incorporating the results of the clinical trials are published, which typically takes several years. One reason for this lag is the inability of a physician to easily identify the most relevant clinical trial from several clinical trials that are applicable to the specific medical condition or diagnosis of a patient, as each clinical trial has different inclusion and exclusion criteria and the standard-of-care varies widely across different regions and countries. Additionally, most clinical trials measure and quantify metrics different, making it difficult for a physician to easily generalize the results of the clinical trials and apply the results of the clinical trials for clinical decision making in routine clinical practice of a patient.

### BRIEF SUMMARY OF THE INVENTION

In accordance with one or more embodiments, systems and methods for mapping a patient to one or more clinical trials are provided. Patient data of a patient is received and encoded into a patient model of the patient. Synthetic patients are generated for each clinical trial in a set of clinical trials based on characteristics of participants in that clinical trial. The patient model of the patient is compared to each of the synthetic patients to identify synthetic patients matching the patient model. The patient is mapped to one or more clinical trials in the set of clinical trials based on the matching synthetic patients.

In one embodiment, the patient is mapped to the one or more clinical trials by, for each particular clinical trial in the set of clinical trials, determining a number of the matching synthetic patients for the particular clinical trial, comparing the number of the matching synthetic patients for the particular clinical trial with a threshold, and mapping the patient to the particular clinical trial where the number of the matching synthetic patients satisfies the threshold.

In one embodiment, the patient is mapped to the one or more clinical trials by, for each particular clinical trial in the set of clinical trials, by determining a matching quality weighting for each of the matching synthetic patients for the particular clinical trial based on the comparison of the patient model to each of the synthetic patient, determining a sum of the matching quality weights for the matching synthetic patients, comparing the sum of the matching quality weights to a threshold, and mapping the patient to the particular clinical trial where the sum of the matching quality weights satisfies the threshold.

In one embodiment, the synthetic patients are generated by, for each particular clinical trial in the set of clinical trials, determining a distribution for each of a plurality of characteristics of participants in the particular clinical trial and sampling each of the distributions to determine generated characteristics of the synthetic patients.

In one embodiment, the set of clinical trials is automatically selecting based on a primary disease condition.

In one embodiment, the patient model of the patient is compared to each of the synthetic patients by determining a similarity measure between the patient model of the patient and each of the synthetic patients using a similarity criterion and identifying the synthetic patients matching the patient model based on the similarity measure.

In one embodiment, the similarity criterion is determined for each clinical trial in the set of clinical trials based on the characteristics of the participants of that clinical trial.

In one embodiment, the set of clinical trials comprises a set of completed clinical trials.

In one embodiment, the mapping of the patient to the one or more clinical trials is output in a ranked order based on user defined criteria.

These and other advantages of the invention will be apparent to those of ordinary skill in the art by reference to the following detailed description and the accompanying drawings.

### BRIEF DESCRIPTION OF THE DRAWINGS

Figure 1 shows a framework for mapping a patient to a clinical trial, in accordance with one or more embodiments;
Figure 2 shows a method for mapping a patient to a clinical trial, in accordance with one or more embodiments;
Figure 3 shows a table of exemplary baseline characteristics for a clinical trial, in accordance with one or more embodiments; and
Figure 4 shows a high-level block diagram of a computer.

### DETAILED DESCRIPTION

The present invention generally relates to methods and systems for mapping a patient to clinical trials for patient specific clinical decision support. Embodiments of the present invention are described herein to give a visual understanding of such methods and systems. A digital image is often composed of digital representations of one or more objects (or shapes). The digital representation of an object is often described herein in terms of identifying and manipulating the objects. Such manipulations are virtual manipulations accomplished in the memory or other circuitry/hardware of a computer system. Accordingly, is to be understood that embodiments of the present invention may be performed within a computer system using data stored within the computer system.

Embodiments described herein provide for the mapping of a patient to clinical trials, thereby enabling a clinician (e.g., physician or other medical professional) to consider results of the clinical trial for treating the patient. Such mapping is enabled by generating synthetic patients for the clinical trial, which allows for a comparison between the patient and the clinical trial via a patient-to-patient comparison between the patient and the synthetic patients for the clinical trial. Advantageously, embodiments described herein enable faster incorporation of results of clinical trials in routine patient care, thereby providing improved clinical decision making and improved patient care.

As used herein, a clinical trial refers to any evaluation of a medical activity on one or more participants, and is not limited to medical activities formally designated or approved as a clinical trial by a government, an organization, or any other entity. Exemplary medical activities include treatments (e.g., drugs, vaccines, medical devices, interventions), diagnoses, or any other medical activity.

Further, it should be understood that embodiments described herein are generally directed to the mapping of a patient to a completed clinical trial for clinical decision support based on results of the completed clinical trial, and not to matching a patient to an open clinical trial for enrolling the patient as a participant in the open clinical trial.

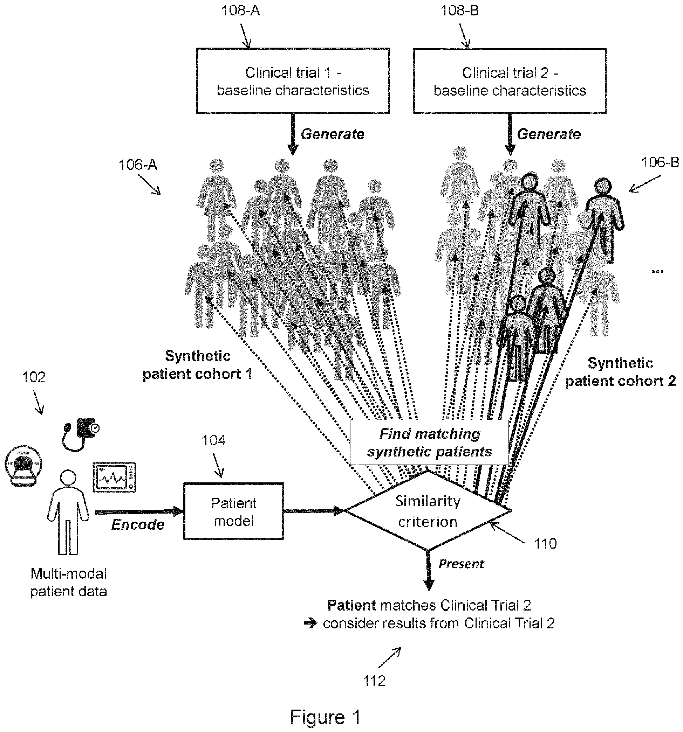

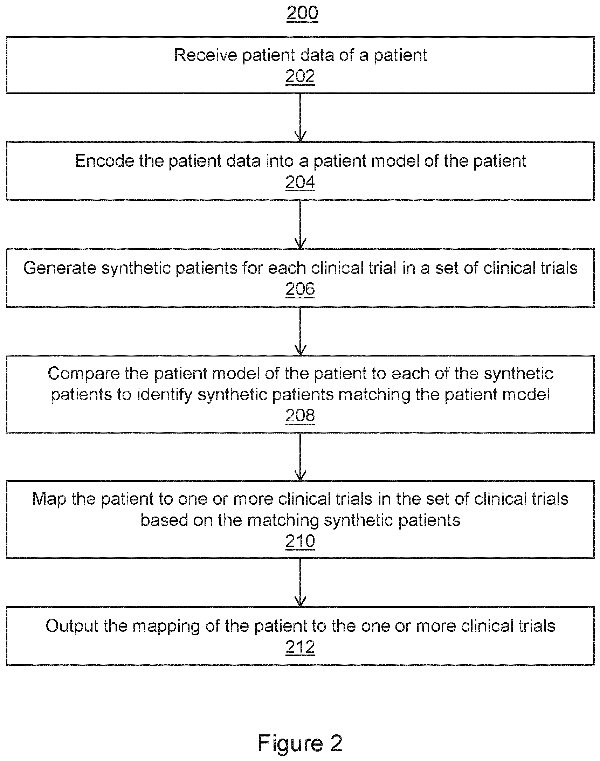

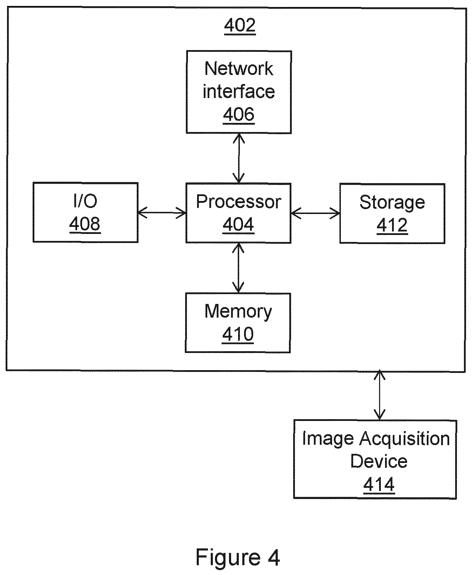

Figure 1 shows a framework 100 for mapping a patient to a clinical trial, in accordance with one or more embodiments. Figure 2 shows a method 200 for mapping a patient to a clinical trial, in accordance with one or more embodiments. Figures 1 and 2 will be described together. The steps of method 200 may be performed using one or more suitable computing devices, such as, e.g., computer 402 of Figure 4.

At step 202, patient data of a patient is received. The patient data may be multi-modal patient data 102 in Figure 1. The patient data may include any data relating to the patient, such as, e.g., medical records, medical imaging data, laboratory data, drug and prescription data, demographic information, etc. In one embodiment, the patient data is retrieved from various disparate data management systems, such as, e.g., an EMR (electronic medical record), an EHR (electronic health record), a PACS (picture archiving and communication system), a LIS (laboratory information system), an RIS (radiology information system), or a CVIS (cardiovascular information system), and combined as integrated data represented hierarchically according to the FHIR (fast healthcare interoperability resources) specification or any other standard for data interoperability.

At step 204, the patient data is encoded into a patient model of the patient. The patient model may be patient model 104 encoded from multi-modal patient data 102 in Figure 1. The patient model may be in any suitable format having a predefined structure. In one embodiment, the patient model is a vector. The patient data may be encoded into the patient model using any dimensionality reduction method.

In one embodiment, the patient data is encoded into the patient model using a trained machine learning network, such as, e.g., an autoencoder. An encoding network of the autoencoder may receive the patient data as input and encode the patient data into low dimensional features, thereby forming a vector of features. The encoding network may be trained, together with a decoding network of the autoencoder, for data reconstruction during a prior offline or training stage.

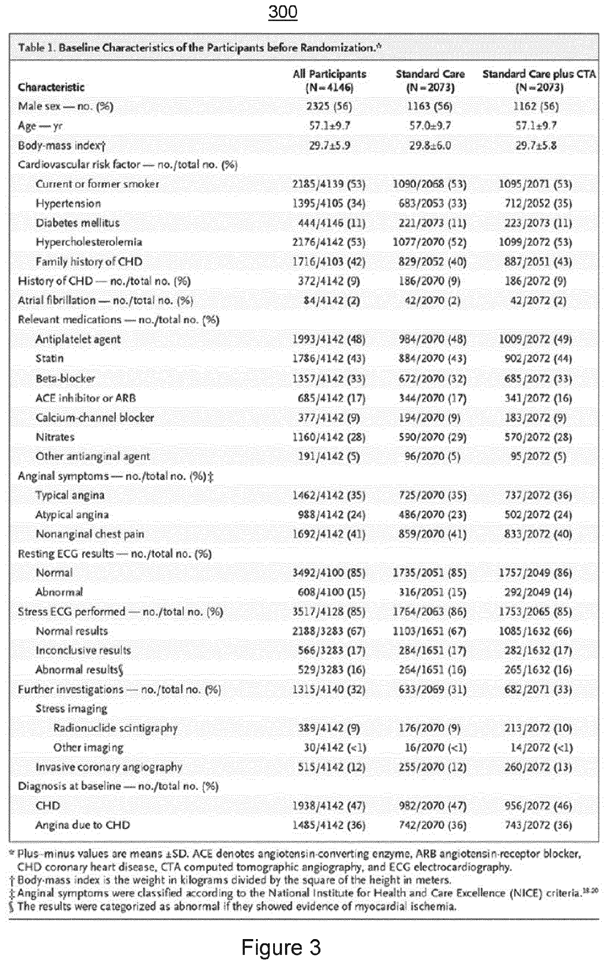

At step 206, synthetic patients for each clinical trial in a set of clinical trials are generated. A synthetic patient is a representation of characteristics generated for a hypothetical patient. The synthetic patients may be generated using baseline characteristics of participants of a particular clinical trial and thus the synthetic patients would be eligible for the particular clinical trial. In one example, the synthetic patients are the synthetic patients in synthetic patient cohort 106-A and 106-B generated from respective baseline characteristics 108-A of clinical trial 1 and baseline characteristics 108-B of clinical trial 2 in Figure 1. The baseline characteristics may include any characteristic of the participants of the clinical trials, such as, e.g., age, weight, height, gender, BMI (body mass index), results of lab tests, symptoms, history, findings from imaging exams, co-morbidities, etc. Figure 3 shows a table 300 of exemplary baseline characteristics of a clinical trial, in accordance with one embodiment.

In one embodiment, the synthetic patients for a clinical trial are generated by determining a distribution for each baseline characteristic (independently or jointly) of the clinical trial and randomly sampling from each distribution to generate characteristics of synthetic patients that fall within the baseline characteristics of the clinical trial. The generated characteristics of the synthetic patients are then encoded into the same predefined format as the patient model to generate the synthetic patients. The generated characteristics of the synthetic patients may be encoded, e.g., using the same methods for encoding the patient data, as described above with respect to step 204.

The set of clinical trials may include one or more clinical trials. In one embodiment, the set of clinical trials are clinical trials of interest selected by a user. The user may define criteria for identifying and/or selecting the clinical trials of interest. In one example, the criteria may include rules defining site-specific constraints, such as, e.g., the non-availability of a particular diagnostic test at an institution. In another embodiment, the set of clinical trials may be automatically selected based on the primary disease condition of the clinical trials. The primary disease condition of the clinical trials may be identified using NLP (natural language processing) or any other suitable technique. User defined criteria may also be employed to further exclude clinical trials (e.g., exclusion of clinical trials involving untrusted clinical sites) or to rank clinical trials. The rank of the clinical trials may be used to prioritize the clinical trials.

At step 208, the patient model of the patient is compared to each of the synthetic patients to identify synthetic patients matching the patient model. The patient model may be compared to each of the synthetic patients based on a similarity measure determined using any suitable similarity criterion, such as, e.g., a pre-defined or learned similarity metric or distance metric. A synthetic patient is identified as matching the patient model when the similarity measure between the patient model and the synthetic patient is within a threshold value. As shown in Figure 1, similarity criterion 110 is applied to measure a similarity between patient model 104 and synthetic patients in synthetic patient cohorts 106-A and 106-B, and synthetic patients matching patient model 104 are identified (in bold) in synthetic patient cohort 106-B.

In one embodiment, the similarity criterion is determined based on the patient model and the synthetic patients (i.e., the representation of the patient data and the synthetic patient). In one example, the patient model is a vector, where each component is a numerical representation of specific patient data (e.g., patient age, sex (male=0, female=1, other=2, ...), lab value, diagnosis A (yes/no), diagnosis B (yes/no), comorbidity C (yes/no), etc.). The synthetic patients generated based on baseline characteristics have a same representation as the patient model. Accordingly, the similarity criterion may be the distance between the patient model vector and the synthetic patient vector. The distance may be a Euclidean distance, a weighted distance, or a more any other suitable distance metric.

In one embodiment, the similarity criterion is different for each clinical trial. For example, as shown in Figure 1, similarity criterion 110 may be different for synthetic patient cohort 106-A of clinical trial 1 and synthetic patient cohort 106-B of clinical trial 2. Similarity criterion 110 for each clinical trial may be determined based on the baseline characteristics of each clinical trial.

At step 210, the patient is mapped to one or more clinical trials in the set of clinical trials based on the matching synthetic patients.

In one embodiment, a number of matching synthetic patients are determined for each clinical trial in the set of clinical trials and the number is compared with a threshold value. The patient is mapped to each clinical trial in the set of clinical trials that has a number of matching synthetic patients that satisfies (e.g., greater than or equal to) the threshold value. As shown in Figure 1, the patient matches four synthetic patients in synthetic patient cohort 106-B and therefore matches clinical trial 2.

In one embodiment, each synthetic patient is associated with a matching quality weighting determined based on its similarity to the patient model (as determined using the similarity criterion at step 208). The matching quality weighting for matching synthetic patients are summed and the sum is compared to a threshold value. The patient is mapped to each clinical trial that has a sum of matching quality weightings for the matching synthetic patients that satisfies (e.g., greater than or equal to) the threshold value.

At step 212, the mapping of the patient to the one or more clinical trials is output. For example, the mapping of the patient to the one or more clinical trials can be output by displaying the mapping of the patient to the one or more clinical trials on a display device of a computer system, storing the mapping of the patient to the one or more clinical trials on a memory or storage of a computer system, or by transmitting the mapping of the patient to the one or more clinical trials to a remote computer system. An exemplary output is shown in Figure 1, where an output 112 is presented to a user indicating that the patient matches clinical trial 2 and that the user should consider results from clinical trial 2 for treating the patient.

Advantageously, embodiments described herein automatically map a patient to one or more completed clinical trials, thereby positioning the patient in the context of past clinical evidence from the one or more completed clinical trials. Embodiments described herein facilitate patient specific clinical decision support with the latest evidence from clinical trials.

In one embodiment, where a plurality of clinical trials are mapped to the patient, the plurality of clinical trials may be output (e.g., presented) in an ranked order determined based on user defined criteria.

In one embodiment, before the patient model of the patient is compared to each of the synthetic patients, the patient model and the synthetic patients are projected to a latent space and the projected patient model and synthetic patients are compared to identify matching synthetic patients at step 208.

In one embodiment, the comparison between the patient model and the synthetic patients at step 208 is further used to identifying discriminating factors between the patient and the clinical trials. For example, such discriminating factors may be used to identify the factors that differentiate the patient from patients of certain clinical trials, to identify the factors that result in the patient not being a fit to certain clinical trials, or to identify the factors representing information that is needed to determine whether a patient fits certain clinical trials. Accordingly, the identified discriminating factors may be utilized to automatically suggest a certain course of action (e.g., additional tests) to reduce the uncertainty of whether the patient matches a clinical trial. In one example of identifying discriminating factors, a similarity criteria comprises multiple terms, each describing one of the baseline characteristics. The terms in the similarity criterion with the highest minimal dissimilarity between the patient and all synthetic patients may be identified as discriminating factors.

Systems, apparatuses, and methods described herein may be implemented using digital circuitry, or using one or more computers using well-known computer processors, memory units, storage devices, computer software, and other components. Typically, a computer includes a processor for executing instructions and one or more memories for storing instructions and data. A computer may also include, or be coupled to, one or more mass storage devices, such as one or more magnetic disks, internal hard disks and removable disks, magneto-optical disks, optical disks, etc.

Systems, apparatus, and methods described herein may be implemented using computers operating in a client-server relationship. Typically, in such a system, the client computers are located remotely from the server computer and interact via a network. The client-server relationship may be defined and controlled by computer programs running on the respective client and server computers.

Systems, apparatus, and methods described herein may be implemented within a network-based cloud computing system. In such a network-based cloud computing system, a server or another processor that is connected to a network communicates with one or more client computers via a network. A client computer may communicate with the server via a network browser application residing and operating on the client computer, for example. A client computer may store data on the server and access the data via the network. A client computer may transmit requests for data, or requests for online services, to the server via the network. The server may perform requested services and provide data to the client computer(s). The server may also transmit data adapted to cause a client computer to perform a specified function, e.g., to perform a calculation, to display specified data on a screen, etc. For example, the server may transmit a request adapted to cause a client computer to perform one or more of the steps or functions of the methods and workflows described herein, including one or more of the steps or functions of Figures 1-2. Certain steps or functions of the methods and workflows described herein, including one or more of the steps or functions of Figures 1-2, may be performed by a server or by another processor in a network-based cloud-computing system. Certain steps or functions of the methods and workflows described herein, including one or more of the steps of Figures 1-2, may be performed by a client computer in a network-based cloud computing system. The steps or functions of the methods and workflows described herein, including one or more of the steps of Figures 1-2, may be performed by a server and/or by a client computer in a network-based cloud computing system, in any combination.

Systems, apparatus, and methods described herein may be implemented using a computer program product tangibly embodied in an information carrier, e.g., in a non-transitory machine-readable storage device, for execution by a programmable processor; and the method and workflow steps described herein, including one or more of the steps or functions of Figures 1-2, may be implemented using one or more computer programs that are executable by such a processor. A computer program is a set of computer program instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

A high-level block diagram of an example computer 402 that may be used to implement systems, apparatus, and methods described herein is depicted in Figure 4. Computer 402 includes a processor 404 operatively coupled to a data storage device 412 and a memory 410. Processor 404 controls the overall operation of computer 402 by executing computer program instructions that define such operations. The computer program instructions may be stored in data storage device 412, or other computer readable medium, and loaded into memory 410 when execution of the computer program instructions is desired. Thus, the method and workflow steps or functions of Figures 1-2 can be defined by the computer program instructions stored in memory 410 and/or data storage device 412 and controlled by processor 404 executing the computer program instructions. For example, the computer program instructions can be implemented as computer executable code programmed by one skilled in the art to perform the method and workflow steps or functions of Figures 2-4. Accordingly, by executing the computer program instructions, the processor 404 executes the method and workflow steps or functions of Figures 1-2. Computer 402 may also include one or more network interfaces 406 for communicating with other devices via a network. Computer 402 may also include one or more input/output devices 408 that enable user interaction with computer 402 (e.g., display, keyboard, mouse, speakers, buttons, etc.).

Processor 404 may include both general and special purpose microprocessors, and may be the sole processor or one of multiple processors of computer 402. Processor 404 may include one or more central processing units (CPUs), for example. Processor 404, data storage device 412, and/or memory 410 may include, be supplemented by, or incorporated in, one or more application-specific integrated circuits (ASICs) and/or one or more field programmable gate arrays (FPGAs).

Data storage device 412 and memory 410 each include a tangible non-transitory computer readable storage medium. Data storage device 412, and memory 410, may each include high-speed random access memory, such as dynamic random access memory (DRAM), static random access memory (SRAM), double data rate synchronous dynamic random access memory (DDR RAM), or other random access solid state memory devices, and may include non-volatile memory, such as one or more magnetic disk storage devices such as internal hard disks and removable disks, magneto-optical disk storage devices, optical disk storage devices, flash memory devices, semiconductor memory devices, such as erasable programmable read-only memory (EPROM), electrically erasable programmable read-only memory (EEPROM), compact disc read-only memory (CD-ROM), digital versatile disc read-only memory (DVD-ROM) disks, or other non-volatile solid state storage devices.

Input/output devices 408 may include peripherals, such as a printer, scanner, display screen, etc. For example, input/output devices 408 may include a display device such as a cathode ray tube (CRT) or liquid crystal display (LCD) monitor for displaying information to the user, a keyboard, and a pointing device such as a mouse or a trackball by which the user can provide input to computer 402.

An image acquisition device 414 can be connected to the computer 402 to input image data (e.g., medical images) to the computer 402. It is possible to implement the image acquisition device 414 and the computer 402 as one device. It is also possible that the image acquisition device 414 and the computer 402 communicate wirelessly through a network. In a possible embodiment, the computer 402 can be located remotely with respect to the image acquisition device 414.

Any or all of the systems and apparatus discussed herein may be implemented using one or more computers such as computer 402.

One skilled in the art will recognize that an implementation of an actual computer or computer system may have other structures and may contain other components as well, and that Figure 4 is a high level representation of some of the components of such a computer for illustrative purposes.

The foregoing Detailed Description is to be understood as being in every respect illustrative and exemplary, but not restrictive, and the scope of the invention disclosed herein is not to be determined from the Detailed Description, but rather from the claims as interpreted according to the full breadth permitted by the patent laws. It is to be understood that the embodiments shown and described herein are only illustrative of the principles of the present invention and that various modifications may be implemented by those skilled in the art without departing from the scope and spirit of the invention. Those skilled in the art could implement various other feature combinations without departing from the scope and spirit of the invention.

## Claims

1. A method comprising:
receiving (202) patient data of a patient;
encoding (204) the patient data into a patient model of the patient;
generating (206) synthetic patients for each clinical trial in a set of clinical trials based on characteristics of participants in that clinical trial;
comparing (208) the patient model of the patient to each of the synthetic patients to identify synthetic patients matching the patient model; and
mapping (210) the patient to one or more clinical trials in the set of clinical trials based on the matching synthetic patients.

2. The method of claim 1, wherein mapping the patient to one or more clinical trials in the set of clinical trials based on the matching synthetic patients comprises:
for each particular clinical trial in the set of clinical trials:
determining a number of the matching synthetic patients for the particular clinical trial;
comparing the number of the matching synthetic patients for the particular clinical trial with a threshold; and
mapping the patient to the particular clinical trial where the number of the matching synthetic patients satisfies the threshold.

3. The method of claim 1, wherein mapping the patient to one or more clinical trials in the set of clinical trials based on the matching synthetic patients comprises:
for each particular clinical trial in the set of clinical trials:
determining a matching quality weighting for each of the matching synthetic patients for the particular clinical trial based on the comparison of the patient model to each of the synthetic patient;
determining a sum of the matching quality weights for the matching synthetic patients;
comparing the sum of the matching quality weights to a threshold; and
mapping the patient to the particular clinical trial where the sum of the matching quality weights satisfies the threshold.

4. The method of claim 1, wherein generating synthetic patients for each clinical trial in a set of clinical trials comprises:
for each particular clinical trial in the set of clinical trials:
determining a distribution for each of a plurality of characteristics of participants in the particular clinical trial; and
sampling each of the distributions to determine generated characteristics of the synthetic patients.

5. The method of claim 1, wherein generating synthetic patients for each clinical trial in a set of clinical trials comprises:
automatically selecting the set of clinical trials based on a primary disease condition.

6. The method of claim 1, wherein comparing the patient model of the patient to each of the synthetic patients to identify synthetic patients matching the patient model comprises:
determining a similarity measure between the patient model of the patient and each of the synthetic patients using a similarity criterion; and
identifying the synthetic patients matching the patient model based on the similarity measure.

7. The method of claim 6, further comprising:
determining the similarity criterion for each clinical trial in the set of clinical trials based on the characteristics of the participants of that clinical trial.

8. The method of claim 1, wherein the set of clinical trials comprises a set of completed clinical trials.

9. The method of claim 1, further comprising:
outputting (212) the mapping of the patient to the one or more clinical trials in a ranked order based on user defined criteria.

10. An apparatus comprising:
means for receiving (202) patient data of a patient;
means for encoding (204) the patient data into a patient model of the patient;
means for generating (206) synthetic patients for each clinical trial in a set of clinical trials based on characteristics of participants in that clinical trial;
means for comparing (208) the patient model of the patient to each of the synthetic patients to identify synthetic patients matching the patient model; and
means for mapping (210) the patient to one or more clinical trials in the set of clinical trials based on the matching synthetic patients.

11. The apparatus of claim 10, wherein the means for mapping the patient to one or more clinical trials in the set of clinical trials based on the matching synthetic patients comprises:
means for determining a number of the matching synthetic patients for each clinical trial in the set of clinical trials;
means for comparing the number of the matching synthetic patients for each clinical trial with a threshold; and
means for mapping the patient to each clinical trial where the number of the matching synthetic patients for that clinical trial satisfies the threshold.

12. The apparatus of claim 10, wherein the means for mapping the patient to one or more clinical trials in the set of clinical trials based on the matching synthetic patients comprises:
means for determining a matching quality weighting for each of the matching synthetic patients for each clinical trial in the set of clinical trials based on the comparison of the patient model to each of the synthetic patient;
means for determining a sum of the matching quality weights for the matching synthetic patients for each clinical trial in the set of clinical trials;
means for comparing the sum of the matching quality weights for the matching synthetic patients for each clinical trial to a threshold; and
means for mapping the patient to each clinical trial where the sum of the matching quality weights for the matching synthetic patients for that clinical trial satisfies the threshold.

13. The apparatus of claim 10, wherein the means for generating synthetic patients for each clinical trial in a set of clinical trials comprises:
means for determining a distribution for each of a plurality of characteristics of participants for each clinical trial in the set of clinical trials; and
means for sampling each of the distributions to determine generated characteristics of the synthetic patients for each clinical trial.

14. The apparatus of claim 10, wherein the means for generating synthetic patients for each clinical trial in a set of clinical trials comprises:
means for automatically selecting the set of clinical trials based on a primary disease condition.

15. A non-transitory computer readable medium storing computer program instructions, the computer program instructions when executed by a processor cause the processor to perform operations comprising:
receiving (202) patient data of a patient;
encoding (204) the patient data into a patient model of the patient;
generating (206) synthetic patients for each clinical trial in a set of clinical trials based on characteristics of participants in that clinical trial;
comparing (208) the patient model of the patient to each of the synthetic patients to identify synthetic patients matching the patient model; and
mapping (210) the patient to one or more clinical trials in the set of clinical trials based on the matching synthetic patients.

16. The non-transitory computer readable medium of claim 15, wherein mapping the patient to one or more clinical trials in the set of clinical trials based on the matching synthetic patients comprises:
for each particular clinical trial in the set of clinical trials:
determining a matching quality weighting for each of the matching synthetic patients for the particular clinical trial based on the comparison of the patient model to each of the synthetic patient;
determining a sum of the matching quality weights for the matching synthetic patients;
comparing the sum of the matching quality weights to a threshold; and
mapping the patient to the particular clinical trial where the sum of the matching quality weights satisfies the threshold.

17. The non-transitory computer readable medium of claim 15, wherein comparing the patient model of the patient to each of the synthetic patients to identify synthetic patients matching the patient model comprises:
determining a similarity measure between the patient model of the patient and each of the synthetic patients using a similarity criterion; and
identifying the synthetic patients matching the patient model based on the similarity measure.

18. The non-transitory computer readable medium of claim 17, the operations further comprising:
determining the similarity criterion for each clinical trial in the set of clinical trials based on the characteristics of the participants of that clinical trial.

19. The non-transitory computer readable medium of claim 15, wherein the set of clinical trials comprises a set of completed clinical trials.

20. The non-transitory computer readable medium of claim 15, further comprising:
outputting (212) the mapping of the patient to the one or more clinical trials in a ranked order based on user defined criteria.
